# EUROPÄISCHE PATENTSCHRIFT

(11) **EP 2 508 893 B1**
(45) Veröffentlichungstag und Bekanntmachung des Hinweises auf die Patenterteilung: **26.04.2017**
(21) Anmeldenummer: 12160688.3
(22) Anmeldetag: 22.03.2012
(51) Int. Cl.: G01N 33/86, C12Q 1/56

(54) **Kontrollen und Kit für Thrombozytenaktivitätstests**
Control sample and kit for platelet activity tests
Échantillon contrôle et kit pour des tests d'activité des plaquettes

(30) Priorität: 04.04.2011 EP 11160934
(43) Veröffentlichungstag der Anmeldung: 10.10.2012
(73) Patentinhaber: Siemens Healthcare Diagnostics Products GmbH, 35041 Marburg (DE)
(72) Erfinder: Rechner, Andreas, Dr., 35041 Marburg (DE); Zander, Norbert, Dr., 35039 Marburg (DE)

(56) Entgegenhaltungen:
- EP-A1- 1 544 621
- EP-A1- 1 850 134
- WO-A2-2005/007868
- WO-A2-2006/020773
- US-A1- 2006 034 809
- US-A1- 2007 243 632

## Beschreibung

Die vorliegende Erfindung liegt auf dem Gebiet der Gerinnungsdiagnostik und betrifft ein Kit sowie ein Verfahren zur Herstellung von Kontrollen zur Verwendung bei Testverfahren zur Bestimmung der Thrombozytenfunktion. Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Thrombozyten (Blutplättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik sind verschiedene Verfahren und Systeme bekannt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Ein bekanntes Verfahren zur Untersuchung der Thrombozytenfunktion ist die Blutungszeitbestimmung. Es handelt sich dabei um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen schwer standardisierbaren, grob informativen Test, der vor allem in Notfallsituationen angewendet wird, um einen Überblick über die primäre Hämostase zu erhalten. Die Einnahme von Thrombozytenaggregationshemmern führt zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

In vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein) oder Serotonin.

Im Stand der Technik sind verschiedene Verfahren zur Untersuchung der Thrombozytenfunktion in vitro bekannt.

Bei der Lichttransmissionsaggregometrie, die auch als Plättchenaggregation nach Born bezeichnet wird, wird die Aggregationsfähigkeit der Thrombozyten im plättchenreichen Plasma in Gegenwart von aggregationsinduzierenden Substanzen in einem Aggregometer photometrisch gemessen. Durch die Aggregatbildung wird die Lichtdurchlässigkeit der PRP-Probe erhöht, so dass durch die Messung der Lichtdurchlässigkeit z.B. die Geschwindigkeit der Aggregatbildung bestimmt werden kann. Mit Hilfe der Lichttransmissionsaggregometrie können auch therapeutische Effekte von medikamentös eingesetzten Thrombozytenaggregationshemmern erfasst werden. Ein Nachteil der Lichttransmissionsaggregometrie ist, dass ausschließlich plättchenreiches Plasma als Probenmaterial verwendet werden kann. Plättchenreichem Plasma fehlen nicht nur wichtige Bestandteile des Blutes, wie z.B. die roten und weißen Blutkörperchen, sondern es erfordert außerdem eine zeitaufwändige und fehleranfällige Probenvorbereitung.

Das VerifyNow^{®} System (Accumetrics) ist eine Weiterentwicklung der Lichttransmissionsaggregometrie, die die Untersuchung der Thrombozytenfunktion in Vollblutproben ermöglicht. In diesem System wird die Aggregationsreaktion der Thrombozyten durch die Zugabe von Fibrinogen-beschichteten Mikropartikeln verstärkt. Reagenzien und Kits für die Durchführung dieses Verfahrens sind in der WO 2005/007868 A2 beschrieben.

Ein anderes Testprinzip zur Bestimmung der Thrombozytenfunktion ist das sogenannte Platelet Function Analyzer System, kurz PFA-System (PFA-100^{®}, PFA-200, Siemens Healthcare Diagnostics Products GmbH, Marburg, Deutschland). Mit Hilfe des PFA-Systems wird in Vollblutproben die primäre Hämostase unter Flussbedingungen und damit in Gegenwart von hohen Scherkräften gemessen.

Zur Simulation der Flussbedingungen und der Scherkräfte, wie sie in kleineren arteriellen Blutgefäßen herrschen, wird in einer PFA-Messzelle, die in ein PFA-Analysegerät eingesetzt wird, ein Unterdruck von etwa -40mbar erzeugt, und das Citratvollblut, dass sich in einem Probenreservoir befindet, strömt durch eine Kapillare, die einen Durchmesser von etwa 200 µm hat. Die Kapillare mündet in eine Messkammer, die mit einem Trennelement, z.B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Es werden üblicherweise Messzellen verwendet, die mit einer Membran ausgestattet sind, die mit Kollagen (Kol) und zusätzlich entweder mit ADP oder mit Epinephrin (Epi) beschichtet ist. Andere Messzellen, die insbesondere zur Bestimmung von Antithrombotika aus der Gruppe der P2Y(12)-Antagonisten, wie z.B. von Clopidogrel, geeignet sind, sind mit einer Membran ausgestattet, die ADP und Prostaglandin E1 enthält (EP-A1-1850134).

Ein anderes Testprinzip zur Bestimmung der Thrombozytenfunktion ist das Multiplate^{®} System (Verum Diagnostica GmbH, München, Deutschland). Mit Hilfe des Multiplate-Systems wird in Vollblutproben die primäre Hämostase basierend auf der Impedanzaggregometrie gemessen. Dazu werden zwei Sensoreinheiten, die je aus zwei parallel angeordneten Sensordrähten bestehen und die in einer speziellen Messzelle angeordnet sind, in die Vollblutprobe eingetaucht. Durch die Zugabe von Thrombozytenaktivatoren, wie z.B. ADP, Kollagen oder Arachidonsäure, wird die Aggregation der Thrombozyten induziert. Die Thrombozyten heften sich an der Oberfläche der Sensordrähte an, wodurch sich der elektrische Widerstand zwischen den Sensordrähten erhöht. Die gemessene Erhöhung des elektrischen Widerstandes korreliert mit der Thrombozytenfunktion.

Bislang ist es nicht gelungen, ein funktionsfähiges und langzeitstabiles Kontroll- oder Standardmaterial herzustellen, das für die Kontrolle oder Standardisierung von Thromboyzentenfunktionstesten geeignet ist, die die Thrombozytenfunktion in Vollblut testen.

US 4,338,564 und US 4,358,394 beschreiben Verfahren zur Herstellung von hämatologischen Kontrollmaterialien, die sich zur Verwendung in der Zytometrie eignen, weil in diesen Präparationen insbesondere Zahl, Form und Größe der Blutzellen gewährleistet sind. Für die Untersuchung der Thrombozytenfunktion sind diese Präparationen jedoch ungeeignet, weil die Zellen mit Fixierungsmitteln und anderen Zellmembran-verändernden Substanzen behandelt sind.

Der vorliegenden Erfindung lag daher die Aufgabe zugrunde, Kontrollmaterial für Vollblut-Thromboyzentenfunktionsteste bereitzustellen.

Die Aufgabe wird dadurch gelöst, dass ein lagerfähiges Kit bereit gestellt wird, das es dem Verwender eines Thromboyzentenfunktionstests ermöglicht, auf einfache Weise standardisierte Kontrollen herzustellen.

Die Erfindung betrifft daher ein Kit gemäß einem der Ansprüche 1 bis 3 zur Herstellung von Kontrollen für ein Verfahren zur Bestimmung der Thrombozytenfunktion, wobei das Kit mindestens ein erstes Lyophilisat zur Herstellung einer ersten Kontrolle mit normaler Thrombozytenfunktion und ein zweites Lyophilisat zur Herstellung einer zweiten Kontrolle mit abnormal verringerter Thrombozytenfunktion enthält.

Zur Herstellung der Kontrollen wird jedes Lyophilisat in Vollblut, welches eine normale Thrombozytenfunktion aufweist, gelöst.

Jedes Lyophilisat eines erfindungsgemäßen Kits besteht mindestens aus einer Puffersubstanz zur Aufrechterhaltung eines physiologischen pH Wertes und einem Hilfsstoff für die Lyophilisierung.

Der physiologische pH Wert von Blut liegt üblicherweise zwischen etwa 7.35 und 7.45. Bevorzugte Puffersubstanzen zur Aufrechterhaltung eines physiologischen pH Wertes sind HEPES (2-(4-(2-Hydroxyethyl)- 1-piperazinyl)-ethansulfonsäure), PBS (Phosphatgepufferte Salzlösung), OVB (Owren's Veronal Puffer), MES (2-(N-Morpholino)ethansulfonsäure), PIPES (Piperazin-N,N'-bis(2-ethanesulfonsäure), TAPS (N-Tris(hydroxymethyl)methyl-3-aminopropansulfonsäure), CHES (2-(Cyclohexylamino)ethansulfonsäure), CAPS (N-cyclohexyl-3-aminopropansulfonsäure), Tris (Tris(hydroxymethyl)-aminomethan) oder MOPS (3-(N-morpholino)propansulfonsäure).

Bevorzugte Hilfsstoffe für die Lyophilisierung sind Substanzen der Gruppe der Disaccharide, unter anderem umfassend Saccharose, Lactose, Trehalose und Maltose, und der Gruppe der Alkohole, bevorzugt Polyethylenglykol, Mannitol und Sorbitol. Diese Substanzen haben kryo- bzw. lyoprotektive Wirkung, d.h. sie schützen andere Bestandteile der Präparation während des Einfrierens und Gefriertrocknens.

Das Lyophilisat zur Herstellung einer Kontrolle mit normaler Thrombozytenfunktion ist frei von Substanzen, die die Thrombozytenfunktion beeinflussen.

Ein Lyophilisat zur Herstellung einer Kontrolle mit abnormal verringerter Thrombozytenfunktion enthält zusätzlich mindestens einen direkten Inhibitor der Thrombozytenaggregation aus der Gruppe Tirofiban, Abxicimab, Eptifibatid, Acetylsalicylsäure, MRS 2395, AR-C66096, Cangrelor, Ticagrelor, Cilostazol, Dipyridamol, Prostaglandin El (PGE1), Prostacyclin, Iloprost, Cicaprost, Forskolin, 2-MeSAMP (2-Methylthioadenosin-5'-monophospat), C1330-7 (N1-(6-Ethoxy-3,3-benzothiazol-2-yl-2-(7-ethoxy-4-hydroxy-2,2-dioxo-2H-2-6benzo[4,5][1,3]thiazolo [2,3-c][1,2,4]thiadiazin-3-yl)-2-oxo-1-ethansulfonamid), MRS 2179 (2'-Deoxy-N6-methyladenosin 3',5'-diphosphat-Diammoniumsalz), MRS 2279 ((N)-Methanocarba-N6-methyl-2-chloro-2'-deoxyadenosin-3',5'-bisphosphat), MRS 2500 (2-Iodo-N6-methyl-(N)-methanocarba-2'-deoxyadenosin-3',5'-bisphosphat), A2P5P (Adenosin-2',5'-bisphosphat), A3P5P (Adenosin-3',5'-bisphosphat) und A3P5PS (Adenosin-3'-phosphat,5'-phosphosulfat). Unter einem direkten Inhibitor der Thrombozytenaggregation ist eine Substanz zu verstehen, die eine direkte Wirkung auf die Thrombozytenfunktion hat, wie beispielsweise durch Blockade der thrombozytären ADP-Rezeptoren, Hemmung der thrombozytären Cyclooxygenase (COX) oder Blockade der thrombozytären des GP-IIb/IIIa-Rezeptoren. Direkte Inhibitoren der Thrombozytenaggregation sind zu unterscheiden von den sogenannten Antikoagulanzien, die ihre gerinnungshemmende Wirkung durch die Hemmung von plasmatischen Gerinnungsfaktoren, wie Thrombin oder Faktor Xa, entfalten und dadurch indirekt einen Einfluss auf die Thrombozytenfunktion haben können. Im Gegensatz zu den Antikoagulanzien bewirken direkte Inhibitoren der Thrombozytenaggregation zum Beispiel keine Verlängerung der Gerinnungszeit in einem Gerinnungstest in Plasma.

Ein Lyophilisat zur Herstellung einer Kontrolle mit abnormal verringerter Thrombozytenfunktion enthält mindestens einen direkten Inhibitor der Thrombozytenaggregation in einer solchen Menge, dass eine Kontrolle herstellbar ist, die bevorzugt eine Thrombozytenfunktion von etwa 50 % bis etwa 80 %, besonders bevorzugt von etwa 70 % der normalen Thrombozytenfunktion aufweist.

Ein bevorzugtes Kit enthält mehrere verschiedene Lyophilisate zur Herstellung von Kontrollen mit abnormal verringerter Thrombozytenfunktion. Diese verschiedenen Lyophilisate können denselben direkten Inhibitor der Thrombozytenaggregation enthalten jedoch in unterschiedlicher Menge. Alternativ können die Lyophilisate unterschiedliche direkte Inhibitoren der Thrombozytenaggregation enthalten.

Ein besonders bevorzugtes Testkit enthält Lyophilisate, die zusätzlich humanes Normalplasma enthalten. Dies hat den Vorteil, dass etwaige Mängel an Plasmabestandteilen in der Vollblutprobe, in der das Lyophilisat bei der Herstellung des Kontrollmaterials gelöst wird, ausgeglichen werden. Niedrige Konzentrationen bestimmter Plasmabestandteile, wie z.B. niedrige Konzentrationen des von Willebrand Faktors (VWF), können die Thrombozytenaktivitätsbestimmung beeinflussen. Durch die Zuführung dieser Bestandteile in Form von humanem Normalplasma wird eine Mindestkonzentration aller plasmatischen Bestandteile gewährleistet, so dass eine Stabilisierung der Thrombozytenfunktion in einer Kontrolle und damit eine verbesserte Standardisierung der Testergebnisse erzielt wird. Ein Lyophilisat eines erfindungsgemäßen Testkits enthält humanes Normalplasma bevorzugt in einer solchen Menge, dass eine Kontrolle herstellbar ist, die nach dem Lösen in Vollblut etwa 2 % bis etwa 20% Volumenprozent humanes Normaplasma enthält.

Ein weiteres besonders bevorzugtes Testkit enthält Lyophilisate, die zusätzlich isolierten humanen von Willebrand Faktor (VWF) enthalten. Dies hat den Vorteil, dass etwaige VWF-Mängel in der Vollblutprobe, in der das Lyophilisat bei der Herstellung des Kontrollmaterials gelöst wird, ausgeglichen werden. Dies bewirkt eine Stabilisierung der Thrombozytenfunktion in einer Kontrolle und damit eine verbesserte Standardisierung der Testergebnisse erzielt wird. Ein Lyophilisat eines erfindungsgemäßen Testkits enthält von Willebrand Faktor, bevorzugt isolierten humanen von Willebrand Faktor, bevorzugt in einer solchen Menge, dass eine Kontrolle herstellbar ist, die nach dem Lösen in Vollblut etwa 0,1 bis etwa 10 IU/mL VWF, besonders bevorzugt 0,1 bis etwa 1,0 IU/mL VWF, ganz besonders bevorzugt 0,1 bis etwa 0,5 IU/mL VWF, enthält.

Der Begriff "Lyophilisat" umfasst Endprodukte der Gefriertrockung.

Zur Herstellung der Lyophilisate eines erfindungsgemäßen Kits werden üblicherweise zunächst wässrige Lösungen hergestellt, die die gewünschten Substanzen in den gewünschten Konzentrationen enthalten. Die Lösungen werden in Gefäße, bevorzugterweise in Glasfläschchen, aliquotiert und anschließend gefriergetrocknet. Die wasserfreien, pulverförmigen Rückstände werden dann luftdicht verschlossen und können über mehrere Monate, gegebenfalls sogar Jahre gelagert werden.

Ein erfindungsgemäßes Testkit enthält bevorzugterweise mehrere Gefäße, bevorzugterweise mehrere Glas- oder Kunststofffläschchen, die die verschiedenen Lyophilisate luftdicht abgeschlossen enthalten.

Ein weiterer Gegenstand der vorliegenden Erfindung ist ein Verfahren zur Herstellung von Kontrollen für ein Verfahren zur Bestimmung der Thrombozytenfunktion. Die Herstellung der Kontrollen erfolgt dadurch, dass ein erfindungsgemäßes Kit, welches verschiedene Lyophilisate enthält, verwendet wird. Die verschiedenen Lyophilisate werden in Vollblut mit normaler Thrombozytenfunktion gelöst. Bevorzugterweise handelt es sich bei dem Vollblut um eine frische Vollblutprobe eines gesunden Spenders. Das Vollblut ist bevorzugterweise mit einer Substanz aus der Gruppe Citrat, Hirudin, PPACK (D-phenylalanyl-L-prolyl-L-arginin Chloromethyl Keton), BAPA (Benzylsulfonyl-D-Arg-Pro-4-amidinobenzylamid) und Heparin antikoaguliert.

Die Herstellung der Kontrollen durch Lösen der Lyophilisate in Vollblut erfolgt bevorzugterweise erst beim Anwender eines Verfahrens zur Bestimmung der Thrombozytenfunktion.

### BEISPIELE

Die folgenden Ausführungsbeispiele dienen der Veranschaulichung der Erfindung und sind nicht als Einschränkung zu verstehen.

### BEISPIEL 1: Herstellung eines erfindungsgemäßen Testkits und Herstellung von Kontrollen für ein Verfahren zur Bestimmung der Thrombozytenfunktion

Es wurde ein Testkit für die Herstellung von Kontrollen, die u.a. für die Verwendung bei Thrombozytenfunktionstesten nach dem Testprinzip des sogenannte Platelet Function Analyzer Systems (PFA-100^{®}, PFA-200, Siemens Healthcare Diagnostics Products GmbH, Marburg, Deutschland) geeignet sind, hergestellt.

Mit Hilfe des PFA-Systems wird in Vollblutproben die primäre Hämostase unter Flussbedingungen und damit in Gegenwart von hohen Scherkräften gemessen. Als Maß für die Thrombozytenfunktion wird in diesem System die Zeit gemessen, die bis zum Verschluss einer Membranöffnung in einer speziellen Messzelle benötigt wird (Verschlusszeit). Messzellen, die mit unterschiedlichen Membrantypen ausgestattet sind, ermöglichen die Bestimmung unterschiedlicher Funktionen der Thrombozyten. Es wurden Messzellen verwendet, die mit einer Membran ausgestattet sind, die mit Kollagen (Kol) und mit ADP beschichtet ist (Kol/ADP), oder die mit Kollagen (Kol) und mit Epinephrin (Epi) beschichtet ist (Kol/Epi), oder die mit ADP und Prostaglandin E1 beschichtet ist (INNOVANCE^{®} PFA P2Y).

Es sollte eine Kontrolle hergestellt werden, die mit allen drei Messzellentypen eine normale Thrombozytenfunktion liefert (normale Kontrolle, Level 1). Ferner sollte eine Kontrolle hergestellt werden, die mit allen drei Messzellentypen eine abnormal verringerte Thrombozytenfunktion liefert (abnormale Kontrolle, Level 2). Ferner sollte eine Kontrolle hergestellt werden, die nur mit Kol/Epi-Messzellen eine abnormal verringerte, mit den anderen beiden Messzellentypen jedoch eine normale Thrombozytenfunktion liefert (abnormale Kontrolle Kol/Epi, Level 2). Ferner sollte eine Kontrolle hergestellt werden, die nur mit INNOVANCE^{®} PFA P2Y-Messzellen eine abnormal verringerte, mit den anderen beiden Messzellentypen jedoch eine normale Thrombozytenfunktion liefert (abnormale Kontrolle P2Y, Level 2).

Die erwünschten Thrombozytenfunktionen der verschiedenen Kontrollen sind in Tab. 1 zusammengefasst.

**Tabelle 1: Thrombozytenfunktionen der verschiedenen Kontrollen**

| **PFA-Messzelle** | **Normale Kontrolle (Level 1)** | **Abnormale Kontrolle (Level 2)** | **Abnormale Kontrolle Kol/EPI** | **Abnormale Kontrolle P2Y** |
|---|---|---|---|---|
| | | | **(Level 2)** | **(Level 2)** |
| **Kol/EPI** | Normal | Abnormal | Abnormal | Normal |
| **Kol/ADP** | Normal | Abnormal | Normal | Normal |
| **INNOVANCE PFA P2Y** | Normal | Abnormal | Normal | Abnormal |

In Tab. 2 sind die Substanzen aufgelistet, die zur Herstellung der Lyophilisate verwendet wurden. Zunächst wurden Stammlösungen in den angegebenen Konzentrationen in A. dest. hergestellt.

**Tabelle 2: Stammlösungen der verwendeten Substanzen**

| Substanz | Konzentration der Stammlösung |
|---|---|
| HEPES | 238 g/mol |
| D-Mannitol | 182 g/mol |
| Acetylsalicylsäure (ASS) | 2,5 g/L |
| Tirofiban Hydrochloride | 10 mg/mL |
| Prostaglandin E1 | 5 mg/mL |
| AR-C 66096 | 10 mM |

Für jede Kontrolle wurden in ein Glasfläschchen (GW 5) definierte Mengen der Stammlösungen der gewünschten Substanzen pipettiert. Die Menge der zu pipettierenden Stammlösung wurde so berechnet, dass eine gewünschte Endkonzentration der Substanzen in der fertigen Kontrolle vorlag. Die Kontrollen sollten durch Zugabe eines Volumens von jeweils 2 mL Vollblut hergestellt werden. Die Endkonzentrationen der Substanzen in den verschiedenen fertigen Kontrollen sind in Tab. 3 zusammengefasst.

Die flüssigen Mischungen in den GW 5-Fläschchen wurden anschließend in einer Gefriertrocknungsanlage nach folgendem Programm gefriergetrocknet: vier Stunden gefroren bei -40°C mit 40 % Vakuum, danach 8 Stunden bei +20°C trocknen.

**Tabelle 3: Endkonzentration der Substanzen in 2 mL Vollblut**

| | **Normale Kontrolle (Level 1)** | **Abnormale Kontrolle (Level 2)** | **Abnormale Kontrolle Kol/EPI** | **Abnormale Kontrolle P2Y** |
|---|---|---|---|---|
| | | | **(Level 2)** | **(Level 2)** |
| **Thrombozyten-Inhibitor** | - | 0,1 µg/mL Tirofiban | 10 nM PGE1 + 50 µM ASS | 200 nM AR-C66096 |
| **Puffer** | 5 mM HEPES | 5 mM HEPES | 5 mM HEPES | 5 mM HEPES |
| **Hilfsstoffe** | 0,001 % Mannitol | 0,001 % Mannitol | 0,001 % Mannitol | 0,001 % Mannitol |

Die Lyophilisate der in Tab. 3 beschriebenen Kontrollen wurden in jeweils 2 mL Citratvollblut von einem gesunden Blutspender für etwa 10 min bei Raumtemperatur gelöst.

### BEISPIEL 2: Verwendung erfindungsgemäß hergestellter Kontrollen in einem Verfahren zur Bestimmung der Thrombozytenfunktion im PFA-System

Die Thrombozytenfunktion der gemäß Beispiel 1 hergestellten Kontrollen wurde mit allen dreien oder mit einzelnen PFA-Messzellen in Doppelbestimmung in einem PFA-Analyzer gemessen. Parallel wurden dieselben Vollblutproben in nativem Zustand (d.h. ohne Zugabe von Lyophilisaten) gemessen, die zum Lösen der Lyophilisate verwendet worden waren.

Für die drei verschiedenen Messzellentypen waren vorab Grenzwerte (Cut-offs) für die Verschlusszeit (in Sekunden) bestimmt worden, die die Unterscheidung zwischen einer normalen und einer abnormal verringerten Thrombozytenfunktion erlauben. Proben mit einer abnormal verringerten Thrombozytenfunktion weisen eine velängerte Verschlusszeit auf, die oberhalb des Cut-offs liegt. Die Cut-offs für die verschiedenen Messzellentypen sind in Tab. 4 zusammengefasst.

**Tabelle 4: Cut-offs der drei PFA-Messzellentypen**

| | **Kol/EPI** | **Kol/ADP** | **INNOVANCE PFA P2Y** |
|---|---|---|---|
| **Cut-off** | 165 s | 119 s | 106 s |

### Normale Kontrolle (Level 1) (HEPES + Mannitol)

Die normale Kontrolle (Level 1), die durch Lösen von lyophilisiertem HEPES und Mannitol in Vollblut hergestellt worden war, weist mit allen drei Messzellentypen gegenüber nativen Vollblutproben leicht verlängerte, aber normale Verschlusszeiten auf. In Tab. 5 sind die mittleren Verschlusszeiten von nativen Vollblutproben im Vergleich zu den mittleren Verschlusszeiten einer normalen Kontrolle (Level 1) in den verschiedenen Messzellentypen gezeigt (aliquotierte Vollblutproben von insgesamt 14 gesunden Spendern).

**Tabelle 5: Mittlere Verschlusszeiten der normalen Kontrolle (Level 1)**

| **Messzelle** | **Cut-off** | **Natives Vollblut** | **Normale Kontrolle (Level 1)** |
|---|---|---|---|
| **Kol/Epi** | 165 s | 99 s | 112 s |
| **Kol/ADP** | 119 s | 85 s | 90 s |
| **INNOVANCE PFA P2Y** | 106 s | 64 s | 74 s |

### Abnormale Kontrolle (Level 2) (HEPES + Mannitol + Tirofiban)

Da Tirofiban den aktivierten GPIIb/IIIa-Rezeptor der Thrombozyten blockiert, verhindert es unabhängig von der Aktivierungsart bzw. dem Messzellentyp die Aggregation der Thrombozyten. Erste Untersuchungen wurden daher nur mit der am stärksten aktivierenden Messzelle Kol/ADP durchgeführt. Die abnormale Kontrolle (Level 2), die durch Lösen von lyophilisiertem HEPES, Mannitol und Tirofiban in Vollblut hergestellt worden war, weist mit dem Messzellentyp Kol/ADP gegenüber nativen Vollblutproben und gegenüber normalen Kontrollen (Level 1) deutlich verlängerte, abnormale Verschlusszeiten oberhalb des Cut-offs auf. In Tab. 6 sind die mittleren Verschlusszeiten von nativen Vollblutproben und von normalen Kontrollen (Level 1) im Vergleich zu den mittleren Verschlusszeiten einer abnormalen Kontrolle (Level 2) in Kol/ADP-Messzellen gezeigt (aliquotierte Vollblutproben von insgesamt 14 gesunden Spendern).

**Tabelle 6: Mittlere Kol/ADP-Verschlusszeit der abnormalen Kontrolle (Level 2)**

| **Messzelle** | **Cut-off** | **Natives Vollblut** | **Normale Kontrolle** | **Abnormale Kontrolle** |
|---|---|---|---|---|
| | | | **(Level 1)** | **(Level 2)** |
| **Kol/ADP** | 119 s | 87 s | 93 s | 287 s |

### Abnormale Kontrolle Kol/EPI (Level 2) (HEPES + Mannitol + Acetylsalicylsäure + Prostaglandin E1)

Die Kol/Epi-spezifische abnormale Kontrolle Kol/Epi (Level 2), die durch Lösen von lyophilisiertem HEPES, Mannitol, Acetylsalicylsäure und PGE1 in Vollblut hergestellt worden war, weist mit dem Messzellentyp Kol/Epi gegenüber nativen Vollblutproben und gegenüber normalen Kontrollen (Level 1) deutlich verlängerte, abnormale Verschlusszeiten oberhalb des Cut-offs auf. In Tab. 7 sind die mittleren Verschlusszeiten von nativen Vollblutproben und von normalen Kontrollen (Level 1) im Vergleich zu den mittleren Verschlusszeiten einer abnormalen Kontrolle Kol/Epi (Level 2) in Kol/Epi-Messzellen gezeigt (aliquotierte Vollblutproben von insgesamt 10 gesunden Spendern).

**Tabelle 7: Mittlere Kol/Epi-Verschlusszeit der abnormalen Kontrolle Kol/Epi (Level 2)**

| **Messzelle** | **Cut-off** | **Natives Vollblut** | **Normale Kontrolle (Level 1)** | **Abnormale Kontrolle Kol/Epi (Level 2)** |
|---|---|---|---|---|
| **Kol/Epi** | 165 s | 106 s | 117 s | 279 s |

### Abnormale Kontrolle P2Y (Level 2) (HEPES + Mannitol + AR-C66096)

Die INNOVANCE PFA P2Y-spezifische abnormale Kontrolle P2Y (Level 2), die durch Lösen von lyophilisiertem HEPES, Mannitol und AR-C66096 in Vollblut hergestellt worden war, weist mit dem Messzellentyp INNOVANCE PFA P2Y gegenüber nativen Vollblutproben und gegenüber normalen Kontrollen (Level 1) deutlich verlängerte, abnormale Verschlusszeiten oberhalb des Cut-offs auf (siehe Fig. 4). In Tab. 8 sind die mittleren Verschlusszeiten von nativen Vollblutproben und von normalen Kontrollen (Level 1) im Vergleich zu den mittleren Verschlusszeiten einer abnormalen Kontrolle P2Y (Level 2) in INNOVANCE PFA P2Y-Messzellen gezeigt (aliquotierte Vollblutproben von insgesamt 4 gesunden Spendern).

**Tabelle 8: Mittlere INNOVANCE PFA P2Y-Verschlusszeit der abnormalen Kontrolle P2Y (Level 2)**

| **Messzelle** | **Cut-off** | **Natives Vollblut** | **Normale Kontrolle (Level 1)** | **Abnormale Kontrolle P2Y (Level 2)** |
|---|---|---|---|---|
| **INNOVANCE PFA P2Y** | 106 s | 79 s | 79 s | 300 s |

In Tab. 9 sind die mittleren Verschlusszeiten von nativen Vollblutproben und von den damit erfindungsgemäß hergestellten normalen und abnormalen Kontrollen in den drei verschiedenen PFA-Messzellentypen gezeigt (aliquotierte Vollblutproben von insgesamt 4 gesunden Spendern). Die erfindungsgemäß hergestellten Kontrollen weisen die gewünschten Thrombozytenaktivitäten auf (vergleiche mit Tab. 1).

**Tabelle 9: Mittlere Verschlusszeiten der vier verschiedenen Kontrollen in den drei verschiedenen Messzellentypen**

| **PFA-Messzelle** | **Natives Vollblut** | **Normale Kontrolle (Level 1)** | **Abnormale Kontrolle (Level 2)** | **Abnormale Kontrolle Kol/EPI (Level 2)** | **Abnormale Kontrolle P2Y (Level 2)** |
|---|---|---|---|---|---|
| **Kol/EPI Cut-off: 165 s** | 119 s | 133 s (normal) | 300 s (abnormal) | 282 s (abnormal) | 152 s (normal) |
| **Kol/ADP Cut-off: 119 s** | 100 s | 108 s (normal) | 300 s (abnormal) | 109 s (normal) | 116 s (normal) |
| **INNOVANCE PFA P2Y Cut-off: 106 s** | 79 s | 79 s (normal) | 300 s (abnormal) | 90 s (normal) | 300 s (abnormal) |

## Patentansprüche

1. Kit zur Herstellung von Kontrollen für ein Verfahren zur Bestimmung der Thrombozytenfunktion, wobei das Kit folgende Komponenten enthält:
a. ein erstes Lyophilisat zur Herstellung einer ersten Kontrolle mit normaler Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung; und
b. ein zweites Lyophilisat zur Herstellung einer zweiten Kontrolle mit abnormal verringerter Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung, und
- einem direkten Inhibitor der Thrombozytenaggregation aus der Gruppe Tirofiban, Abxicimab, Eptifibatid, Acetylsalicylsäure, MRS 2395, AR-C66096, Cangrelor, Ticagrelor, Cilostazol, Dipyridamol, Prostaglandin E1, Prostacyclin, Iloprost, Cicaprost, Forskolin, 2-MeSAMP, C1330-7, MRS 2179, MRS 2279, MRS 2500, A2P5P, A3P5P und A3P5PS.

2. Kit zur Herstellung von Kontrollen für ein Verfahren zur Bestimmung der Thrombozytenfunktion, wobei das Kit folgende Komponenten enthält:
a. ein erstes Lyophilisat zur Herstellung einer ersten Kontrolle mit normaler Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung, und
- humanem Normalplasma; und
b. ein zweites Lyophilisat zur Herstellung einer zweiten Kontrolle mit abnormal verringerter Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung, und
- einem direkten Inhibitor der Thrombozytenaggregation aus der Gruppe Tirofiban, Abxicimab, Eptifibatid, Acetylsalicylsäure, MRS 2395, AR-C66096, Cangrelor, Ticagrelor, Cilostazol, Dipyridamol, Prostaglandin E1, Pröstacyclin, Iloprost, Cicaprost, Forskolin, 2-MeSAMP, C1330-7, MRS 2179, MRS 2279, MRS 2500, A2P5P, A3P5P und A3P5PS und
- humanem Normalplasma.

3. Kit zur Herstellung von Kontrollen für ein Verfahren zur Bestimmung der Thrombozytenfunktion, wobei das Kit folgende Komponenten enthält:
a. ein erstes Lyophilisat zur Herstellung einer ersten Kontrolle mit normaler Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung, und
- humanem von Willebrand Faktor; und
b. ein zweites Lyophilisat zur Herstellung einer zweiten Kontrolle mit abnormal verringerter Thrombozytenfunktion, wobei das Lyophilisat aus folgenden Komponenten besteht:
- einer Puffersubstanz zur Aufrechterhaltung eines physiologischen pH Wertes, und
- einem Hilfsstoff für die Lyophilisierung, und
- einem direkten Inhibitor der Thrombozytenaggregation aus der Gruppe Tirofiban, Abxicimab, Eptifibatid, Acetylsalicylsäure, MRS 2395, AR-C66096, Cangrelor, Ticagrelor, Cilostazol, Dipyridamol, Prostaglandin E1, Prostacyclin, Iloprost, Cicaprost, Forskolin, 2-MeSAMP, C1330-7, MRS 2179, MRS 2279, MRS 2500, A2P5P, A3P5P und A3P5PS und
- humanem von Willebrand Faktor.

4. Kit nach einem der vorhergehenden Ansprüche, wobei die Puffersubstanz zur Aufrechterhaltung eines physiologischen pH Wertes ausgewählt ist aus der Gruppe HEPES, PBS, OVB, MES, PIPES, TAPS, CHES, CAPS, Tris und MOPS.

5. Kit nach einem der vorhergehenden Ansprüche, wobei der Hilfsstoff für die Lyophilisierung ein Disaccharid, bevorzugt ein Disaccharid aus der Gruppe Saccharose, Lactose, Trehalose und Maltose, ist.

6. Kit nach einem der vorhergehenden Ansprüche, wobei der Hilfsstoff für die Lyophilisierung ein Alkohol, bevorzugt ein Alkohol aus der Gruppe Polyethylenglykol, Mannitol und Sorbitol, ist.

7. Kit nach einem der vorhergehenden Ansprüche, wobei das zweite Lyophilisat einen direkten Inhibitor der Thrombozytenaggregation in einer Menge enthält, so dass eine Kontrolle herstellbar ist, die eine Thrombozytenfunktion von zwischen etwa 50 % und etwa 80 %, bevorzugterweise von etwa 70 % der normalen Thrombozytenfunktion aufweist.

8. Kit nach einem der vorhergehenden Ansprüche, wobei das Kit ferner mindestens ein weiteres Lyophilisat zur Herstellung mindestens einer weiteren Kontrolle mit abnormal verringerter Thrombozytenfunktion enthält, wobei das mindestens eine weitere Lyophilisat denselben direkten Inhibitor der Thrombozytenaggregation enthält wie das zweite Lyophilisat, jedoch in unterschiedlicher Menge oder wobei das mindestens eine weitere Lyophilisat einen anderen direkten Inhibitor der Thrombozytenaggregation enthält als das zweite Lyophilisat.

9. Verfahren zur Herstellung von Kontrollen für ein Verfahren zur Bestimmung der Thrombozytenfunktion, **dadurch gekennzeichnet, dass** ein Kit gemäß einem der vorhergehenden Ansprüche verwendet wird und wobei jedes der in dem Kit enthaltenen Lyophilisate in Vollblut mit normaler Thrombozytenfunktion gelöst wird.

10. Verfahren nach Anspruch 9, wobei das Vollblut mit normaler Thrombozytenfunktion eine Vollblutprobe eines gesunden Spenders ist.

11. Verfahren nach einem der Ansprüche 9 oder 10, wobei das Vollblut mit Citrat, Hirudin, PPACK, BAPA oder Heparin antikoaguliert ist.

## Claims

1. Kit for preparing controls for a method for determining thrombocyte function, wherein the kit contains the following components:
a. a first lyophilisate for preparing a first control having normal thrombocyte function, wherein the lyophilisate consists of the following components:
- a buffer substance for maintaining a physiological pH, and
- an auxiliary substance for lyophilization, and
b. a second lyophilisate for preparing a second control having abnormally reduced thrombocyte function, wherein the lyophilisate consists of the following components:
- a buffer substance for maintaining a physiological pH, and
- an auxiliary substance for lyophilization, and
- a direct inhibitor of thrombocyte aggregation from the group consisting of tirofiban, abciximab, eptifibatide, acetylsalicylic acid, MRS 2395, AR-C66096, cangrelor, ticagrelor, cilostazol, dipyridamole, prostaglandin E1, prostacyclin, iloprost, cicaprost, forskolin, 2-MeSAMP, C1330-7, MRS 2179, MRS 2279, MRS 2500, A2P5P, A3P5P and A3P5PS.

2. Kit for preparing controls for a method for determining thrombocyte function, wherein the kit contains the following components:
a. a first lyophilisate for preparing a first control having normal thrombocyte function, wherein the lyophilisate consists of the following components:
- a buffer substance for maintaining a physiological pH, and
- an auxiliary substance for lyophilization, and
- normal human plasma; and
b. a second lyophilisate for preparing a second control having abnormally reduced thrombocyte function, wherein the lyophilisate consists of the following components:
- a buffer substance for maintaining a physiological pH, and
- an auxiliary substance for lyophilization, and
- a direct inhibitor of thrombocyte aggregation from the group consisting of tirofiban, abciximab, eptifibatide, acetylsalicylic acid, MRS 2395, AR-C66096, cangrelor, ticagrelor, cilostazol, dipyridamole, prostaglandin E1, prostacyclin, iloprost, cicaprost, forskolin, 2-MeSAMP, C1330-7, MRS 2179, MRS 2279, MRS 2500, A2P5P, A3P5P and A3P5PS and
- normal human plasma.

3. Kit for preparing controls for a method for determining thrombocyte function, wherein the kit contains the following components:
a. a first lyophilisate for preparing a first control having normal thrombocyte function, wherein the lyophilisate consists of the following components:
- a buffer substance for maintaining a physiological pH, and
- an auxiliary substance for lyophilization; and
- human von Willebrand factor; and
b. a second lyophilisate for preparing a second control having abnormally reduced thrombocyte function, wherein the lyophilisate consists of the following components:
- a buffer substance for maintaining a physiological pH, and
- an auxiliary substance for lyophilization, and
- a direct inhibitor of thrombocyte aggregation from the group consisting of tirofiban, abciximab, eptifibatide, acetylsalicylic acid, MRS 2395, AR-C66096, cangrelor, ticagrelor, cilostazol, dipyridamole, prostaglandin E1, prostacyclin, iloprost, cicaprost, forskolin, 2-MeSAMP, C1330-7, MRS 2179, MRS 2279, MRS 2500, A2P5P, A3P5P and A3P5PS and
- human von Willebrand factor.

4. Kit according to any of the preceding claims, wherein the buffer substance for maintaining a physiological pH is selected from the group consisting of HEPES, PBS, OVB, MES, PIPES, TAPS, CHES, CAPS, Tris and MOPS.

5. Kit according to any of the preceding claims, wherein the auxiliary substance for lyophilization is a disaccharide, preferably a disaccharide from the group consisting of sucrose, lactose, trehalose and maltose.

6. Kit according to any of the preceding claims, wherein the auxiliary substance for lyophilization is an alcohol, preferably an alcohol from the group consisting of polyethylene glycol, mannitol and sorbitol.

7. Kit according to any of the preceding claims, wherein the second lyophilisate contains a direct inhibitor of thrombocyte aggregation in such an amount that a control is obtainable which exhibits thrombocyte function of between about 50% and about 80%, preferably about 70%, of normal thrombocyte function.

8. Kit according to any of the preceding claims, wherein the kit additionally contains at least one further lyophilisate for preparing at least one further control having abnormally reduced thrombocyte function, wherein the at least one further lyophilisate contains the same direct inhibitor of thrombocyte aggregation as the second lyophilisate, but in a different amount, or wherein the at least one further lyophilisate contains a different direct inhibitor of thrombocyte aggregation with respect to the second lyophilisate.

9. Method for preparing controls for a method for determining thrombocyte function, **characterized in that** a kit according to any of the preceding claims is used and wherein each of the lyophilisates contained in the kit is dissolved in whole blood having normal thrombocyte function.

10. Method according to Claim 9, wherein the whole blood having normal thrombocyte function is a whole blood sample from a healthy donor.

11. Method according to either Claim 9 or 10, wherein the whole blood is anticoagulated with citrate, hirudin, PPACK, BAPA or heparin.

## Revendications

1. Trousse de préparation de témoins pour un procédé de détermination de la fonction thrombocytaire, la trousse contenant les constituants suivants :
a. un premier lyophilisat de préparation d'un premier témoin ayant une fonction thrombocytaire normal, dans lequel le lyophilisat est constitué des constituants suivants :
- une substance tampon de maintien d'un pH physiologique et
- un adjuvant de lyophilisation et
b. un deuxième lyophilisat de préparation d'un deuxième témoin ayant une fonction thrombocytaire diminuée anormalement, le lyophilisat étant constitué des constituants suivants :
- une substance tampon de maintien d'un pH physiologique et
- un adjuvant de lyophilisation et
- un inhibiteur direct de l'agrégation des plaquettes choisi dans le groupe tirofiban, abxicimab, eptifibatid, acide acétylsalicylique, MRS 2395, AR-C66096, cangrelor, ticagrelor, cilostazol, dipyridamol, prostaglandine E1, Prostacycline, iloprost, cicaproste, forskoline, 2-MeSamp, C1330-7, MRS 2179, MRS 2279, MRS 2500, A2P5P, A3P5P et A3P5PS.

2. Trousse de préparation de témoins pour un procédé de détermination de la fonction thrombocytaire, la trousse contenant les constituants suivants :
a. un premier lyophilisat de préparation d'un premier témoin ayant une fonction thrombocytaire normal, dans lequel le lyophilisat est constitué des constituants suivants :
- une substance tampon de maintien d'un pH physiologique et
- un adjuvant de lyophilisation et
- du plasma humain normal et
b. un deuxième lyophilisat de préparation d'un deuxième témoin ayant une fonction thrombocytaire diminuée anormalement, le lyophilisat étant constitué des constituants suivants :
- une substance tampon de maintien d'un pH physiologique et
- un adjuvant de lyophilisation et
- un inhibiteur direct de l'agrégation des plaquettes choisi dans le groupe tirofiban, abxicimab, eptifibatid, acide acétylsalicylique, MRS 2395, AR-C66096, cangrelor, ticagrelor, cilostazol, dipyridamol, prostaglandine E1, prostacycline, iloprost, cicaproste, forskoline, 2-MeSamp, C1330-7, MRS 2179, MRS 2279, MRS 2500, A2P5P, A3P5P et A3P5PS et
- du plasma humain normal.

3. Trousse de préparation de témoins pour un procédé de détermination de la fonction thrombocytaire, la trousse contenant les constituants suivants :
a. un premier lyophilisat de préparation d'un premier témoin ayant une fonction thrombocytaire normal, dans lequel le lyophilisat est constitué des constituants suivants :
- une substance tampon de maintien d'un pH physiologique et
- un adjuvant de lyophilisation et
- du facteur de Willebrand humain et
b. un deuxième lyophilisat de préparation d'un deuxième témoin ayant une fonction thrombocytaire diminuée anormalement, le lyophilisat étant constitué des constituants suivants :
- une substance tampon de maintien d'un pH physiologique et
- un adjuvant de lyophilisation et
- un inhibiteur direct de l'agrégation des plaquettes choisi dans le groupe tirofiban, abxicimab, eptifibatid, acide acétylsalicylique, MRS 2395, AR-C66096, cangrelor, ticagrelor, cilostazol, dipyridamol, Prostaglandine E1, prostacycline, iloprost, cicaproste, forskoline, 2-MeSamp, C1330-7, MRS 2179, MRS 2279, MRS 2500, A2P5P, A3P5P et A3P5PS et
- du facteur de Willebrand humain.

4. Trousse suivant l'une des revendications précédentes, dans laquelle la substance tampon de maintien d'un pH physiologique est choisie dans le groupe HEPES, PBS, OVB, MES, PIPES, TAPS, CHES, CAPS, Tris et MOPS.

5. Trousse suivant l'une des revendications précédentes, dans laquelle l'adjuvant de lyophilisation est un disaccharide, de préférence un disaccharide choisi dans le groupe saccharose, lactose, tréhalose et maltose.

6. Trousse de préparation de témoin suivant l'une des revendications précédentes, dans laquelle l'adjuvant de lyophilisation est un alcool, de préférence un alcool choisi dans le groupe polyéthylèneglycol, mannitol et sorbitol.

7. Trousse suivant l'une des revendications précédentes, dans laquelle le deuxième lyophilisat contient un inhibiteur direct de l'agrégation des plaquettes en une quantité telle qu'il peut être préparé un témoin qui a une fonction thrombocytaire représentant entre environ 50 % et environ 80 %, de préférence environ 70 %, de la fonction thrombocytaire normale.

8. Trousse suivant l'une des revendications précédentes, dans laquelle la trousse contient en outre au moins un autre lyophilisat de préparation d'au moins un autre témoin ayant une fonction thrombocytaire diminuée anormalement, le au moins autre lyophilisat contenant le même inhibiteur direct de l'agrégation des plaquettes que le deuxième lyophilisat, mais en une quantité différente ou dans lequel le au moins un autre lyophilisat contient un inhibiteur direct de l'agrégation des plaquettes autre que le deuxième lyophilisat.

9. Procédé de préparation de témoins pour un procédé de détermination de la fonction thrombocytaire, **caractérisé en ce que** l'on utilise une trousse suivant l'une des revendications précédentes et on dissout chacun des lyophilisats contenus dans la trousse dans du sang total ayant une fonction thrombocytaire normale.

10. Procédé selon la revendication 9, dans lequel le sang total ayant une fonction thrombocytaire normale est un échantillon de sang total d'un donneur en bonne santé.

11. Procédé selon l'une des revendications 9 ou 10, dans lequel le sang total est anti-coagulé par du citrate, de l'hirudine, du PPACK, du BAPA ou de l'héparine.
